(19) Europäisches Patentamt / European Patent Office / Office européen des brevets

(11) **EP 3 231 427 A1**

(12) **EUROPEAN PATENT APPLICATION**
published in accordance with Art. 153(4) EPC

(43) Date of publication:
**18.10.2017 Bulletin 2017/42**

(51) Int Cl.:
*A61K 31/5025* (2006.01)   *A61K 9/08* (2006.01)
*A61K 9/107* (2006.01)   *A61K 47/18* (2017.01)
*A61K 47/44* (2017.01)   *A61P 3/10* (2006.01)
*A61P 9/10* (2006.01)   *A61P 35/00* (2006.01)
*A61P 43/00* (2006.01)

(21) Application number: **15868436.5**

(22) Date of filing: **10.12.2015**

(86) International application number:
**PCT/JP2015/084618**

(87) International publication number:
**WO 2016/093299 (16.06.2016 Gazette 2016/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.12.2014 JP 2014250377**

(71) Applicant: **Senju Pharmaceutical Co., Ltd.**
**Osaka-shi, Osaka 541-0046 (JP)**

(72) Inventors:
• **MORI, Yasuhiro**
  **Kobe-shi**
  **Hyogo 651-2241 (JP)**
• **YAMAGUCHI, Masazumi**
  **Kobe-shi**
  **Hyogo 651-2241 (JP)**

(74) Representative: **J A Kemp**
  **14 South Square**
  **Gray's Inn**
  **London WC1R 5JJ (GB)**

(54) **AQUEOUS LIQUID AGENT**

(57)    Provided are an aqueous liquid preparation showing improved stability of N-[5-({2-[(cyclopropylcarbonyl)amino]imidazo[1,2-b]pyridazin-6-yl}oxy)-2-methylphenyl]-1,3-dimethyl-1H-pyrazole-5-carboxamide or a salt thereof in the aqueous liquid preparation, a method of stabilizing the compound in an aqueous liquid, and a stabilizer of the compound in an aqueous liquid.

An aqueous liquid preparation containing the above-mentioned compound and ethylenediaminetetraacetic acid (EDTA) or a salt thereof or a hydrate thereof; a stabilizing method of the compound in an aqueous liquid, including adding the compound and EDTA or a salt thereof or a hydrate thereof to the aqueous liquid; a stabilizer of the above-mentioned compound in an aqueous liquid, which contains EDTA or a salt thereof or a hydrate thereof; and a stabilizer of the compound in an aqueous liquid, which contains EDTA or a salt thereof or a hydrate thereof, and castor oil in combination.

**Description**

[Technical Field]

The present invention relates to an aqueous liquid preparation showing improved stability of N-[5-({2-[(cyclopropylcarbonyl)amino]imidazo[1,2-b]pyridazin-6-yl}oxy)-2-methylphenyl]-1,3-dimethyl-1H-pyrazole-5-carboxamide (to be also referred to as compound A in the present specification) and a salt thereof in the aqueous liquid preparation, a method of stabilizing compound A and a salt thereof in an aqueous liquid, and a stabilizer of compound A and a salt thereof in an aqueous liquid.

[Background Art]

It is known that compound A and a salt thereof have a strong kinase inhibitory activity, and are useful for the prophylaxis or treatment of cancer, prophylaxis or treatment of diabetic retinopathy and the like (patent document 1). However, since compound A and a salt thereof are poorly water soluble, an aqueous liquid preparation thereof, particularly stability in an aqueous liquid, has not been specifically studied.

[Document List]

[Patent document]

patent document 1: WO 2008/016192

[SUMMARY OF THE INVENTION]

[Problems to be Solved by the Invention]

The present inventors have studied an aqueous liquid preparation containing compound A or a salt thereof and found that the content of compound A or a salt thereof in the aqueous liquid preparation problematically decreases day by day.
An object of the present invention is to solve such problem as a new finding and provide an aqueous liquid preparation showing improved stability of compound A and a salt thereof in the aqueous liquid preparation (in other words, a decrease in the content of compound A and a salt thereof in the aqueous liquid preparation is suppressed). Another object of the present invention is to provide a method of stabilizing compound A and a salt thereof in an aqueous liquid, and a stabilizer of compound A and a salt thereof in an aqueous liquid.

[Means of Solving the Problems]

The present inventors have conducted intensive studies in an attempt to solve the aforementioned problems and found that compound A and a salt thereof are stabilized in an aqueous liquid preparation (in other words, a decrease in the content of compound A and a salt thereof in an aqueous liquid preparation is suppressed) by adding ethylenediaminetetraacetic acid or a salt thereof or a hydrate thereof. The present inventors have also found that compound A and a salt thereof are stabilized more in an oil-in-water type emulsion added with ethylenediaminetetraacetic acid or a salt thereof or a hydrate thereof. The present inventors have further conducted intensive studies based on the above-mentioned findings and completed the present invention.
Accordingly, the present invention provides the following.

[1] An aqueous liquid preparation comprising N-[5-({2-[(cyclopropylcarbonyl)amino]imidazo[1,2-b]pyridazin-6-yl} oxy)-2-methylphenyl]-1,3-dimethyl-1H-pyrazole-5-carboxamide or a salt thereof, and ethylenediaminetetraacetic acid or a salt thereof or a hydrate thereof (to be also referred to as the aqueous liquid preparation of the present invention).
[2] The preparation of the above-mentioned [1], which is an oil-in-water type emulsion (to be also referred to as the oil-in-water type emulsion of the present invention in the present specification).
[3] The preparation of the above-mentioned [2], wherein an oil component in the oil-in-water type emulsion is castor oil.
[4] The preparation of any of the above-mentioned [1] - [3], which is an eye drop.
[5] A method of stabilizing N-[5-({2-[(cyclopropylcarbonyl)amino]imidazo[1,2-b]pyridazin-6-yl}oxy)-2-methylphenyl]-1,3-dimethyl-1H-pyrazole-5-carboxamide or a salt thereof in an aqueous liquid, comprising adding N-[5-({2-[(cyclopropylcarbonyl)amino]imidazo[1,2-b]pyridazin-6-yl}oxy)-2-methylphenyl]-1,3-dimethyl-1H-pyrazole-5-carboxamide or a salt thereof, and ethylenediaminetetraacetic acid or a salt thereof or a hydrate thereof to the aqueous liquid.

[6] The method of the above-mentioned [5], wherein the aqueous liquid is an oil-in-water type milky liquid.

[7] The method of the above-mentioned [6], wherein an oil component in the oil-in-water type milky liquid is castor oil.

[8] A stabilizer of N-[5-({2-[(cyclopropylcarbonyl)amino]imidazo[1,2-b]pyridazin-6-yl}oxy)-2-methylphenyl]-1,3-dimethyl-1H-pyrazole-5-carboxamide or a salt thereof in an aqueous liquid, comprising ethylenediaminetetraacetic acid or a salt thereof or a hydrate thereof.

[9] A stabilizer of N-[5-({2-[(cyclopropylcarbonyl)amino]imidazo[1,2-b]pyridazin-6-yl}oxy)-2-methylphenyl]-1,3-dimethyl-1H-pyrazole-5-carboxamide or a salt thereof in an aqueous liquid, comprising ethylenediaminetetraacetic acid or a salt thereof or a hydrate thereof, and castor oil in combination.

[Effect of the Invention]

According to the present invention, an aqueous liquid preparation showing improved stability of compound A and a salt thereof in the aqueous liquid preparation can be provided by adding ethylenediaminetetraacetic acid or a salt thereof or a hydrate thereof to the aqueous liquid preparation (preferably oil-in-water type emulsion) containing compound A or a salt thereof.

[Description of Embodiments]

In the present invention, the "aqueous liquid" is a liquid containing water and, for example, water and oil-in-water type milky liquid can be mentioned. The aqueous liquid refers to a liquid containing generally not more than 50 mass %, preferably not more than 25 mass %, of a substance other than water and a continuous aqueous phase.

In the present invention, moreover, the "aqueous liquid preparation" refers to an aqueous liquid in an embodiment of a pharmaceutical preparation containing compound A or a salt thereof, and includes aqueous solution, suspension, emulsion (e.g., oil-in-water type emulsion) and the like.

The aqueous liquid preparation of the present invention contains compound A or a salt thereof.

The content of compound A or a salt thereof in the aqueous liquid preparation of the present invention is generally 0.002 - 2 w/v%, preferably 0.005 - 0.2 w/v%, further preferably 0.005 - 0.1 w/v%, relative to the total amount of the aqueous liquid preparation.

In the present invention, as a salt of compound A, a pharmaceutically acceptable salt can be mentioned, for example, salts with inorganic acids, salts with organic acids, salts with basic or acidic amino acid and the like can be mentioned. Preferable examples of the salts with inorganic acids include salts with hydrochloric acid, hydrobromic acid, nitric acid, sulfuric acid, phosphoric acid and the like. Preferable examples of the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Preferable examples of the salts with basic amino acids include salts with arginine, lysine, ornithine and the like, and preferable examples of the salts with acidic amino acids include salts with aspartic acid, glutamic acid and the like.

The aqueous liquid preparation of the present invention contains ethylenediaminetetraacetic acid or a salt thereof or a hydrate thereof.

In the present invention, examples of the salt of ethylenediaminetetraacetic acid include sodium salt, potassium salt, calcium salt and magnesium salt, with preference given to sodium salt.

In the present invention, examples of the ethylenediaminetetraacetic acid or a salt thereof or a hydrate thereof include ethylenediaminetetraacetic acid disodium salt, and ethylenediaminetetraacetic acid disodium salt dihydrate, with preference given to ethylenediaminetetraacetic acid disodium salt dihydrate.

The content of the ethylenediaminetetraacetic acid or a salt thereof or a hydrate thereof in the aqueous liquid preparation of the present invention is generally 0.001 - 0.2 w/v%, preferably 0.005 - 0.05 w/v%, further preferably 0.01 - 0.03 w/v%, further more preferably 0.01 - 0.02 w/v%, relative to the total amount of the aqueous liquid preparation.

Since the aqueous liquid preparation of the present invention shows a stabilizing effect on compound A and a salt thereof more remarkably in an emulsion by the addition of ethylenediaminetetraacetic acid or a salt thereof or a hydrate thereof, an oil-in-water type emulsion is preferable.

Examples of the oil component in the oil-in-water type emulsion of the present invention include castor oil, rape seed oil, cottonseed oil, soybean oil, corn oil, olive oil, liquid paraffin, medium-chain triglyceride, fatty acid (e.g., ricinoleic acid, oleic acid), and aliphatic alcohol (e.g., oleyl alcohol), with preference given to castor oil. The oil-in-water type emulsion of the present invention containing castor oil as the oil component is superior in that it suppresses a decrease in the content of compound A and a salt thereof in the aqueous liquid preparation over days. Since compound A and a salt thereof show superior solubility, castor oil is preferably used as the oil component.

The content of the oil component of the oil-in-water type emulsion of the present invention is generally 0.1 - 50% w/v%, preferably 0.5 - 20% w/v%, further preferably 1 - 10% w/v%, relative to the total amount of the aqueous liquid preparation.

The oil-in-water type emulsion of the present invention generally contains an emulsifier.

As the emulsifier, surfactants such as a nonionic surfactant having surfactant capability and the like can be blended. Examples of the nonionic surfactant include polyoxyethylene hydrogenated castor oils, polyoxyethylene sorbitan fatty acid ester (e.g., sorbitan polyoxyethylene monooleates, polyoxyethylene sorbitan monolaurates, sorbitan polyoxyethylene monopalmitates, sorbitan polyoxyethylene monostearates etc.) and the like. Examples of the sorbitan polyoxyethylene monooleates include polysorbate 80. As the emulsifier, sorbitan polyoxyethylene monooleates (particularly, polysorbate 80) are preferable.

The content of the emulsifier in the oil-in-water type emulsion of the present invention is generally 20 - 200 w/w%, preferably 30 - 150 w/w%, further preferably 60 - 120 w/w%, relative to the oil component.

The oil-in-water type emulsion of the present invention can contain a buffering agent. Examples of the buffering agent include acetates such as sodium acetate and the like, phosphates such as sodium dihydrogen phosphate, disodium monohydrogen phosphate, potassium dihydrogen phosphate, dipotassium monohydrogen phosphate and the like, amino-acid salts such as epsilon-aminocaproic acid, sodium glutamate and the like, boric acid and a salt thereof, citric acid and a salt thereof and the like, and sodium acetate is preferable.

The content of the buffering agent in the oil-in-water type emulsion of the present invention is generally 0.01 - 1 w/v%, preferably 0.02 - 0.5 w/v%, further preferably 0.05 - 0.2 w/v%, relative to the total amount of the aqueous liquid preparation.

The oil-in-water type emulsion of the present invention can contain an isotonicity agent. Examples of the isotonicity agent include sodium chloride, glycerin (e.g., concentrated glycerin), propylene glycol, glucose, mannitol, sorbitol and the like, with preference given to glycerin (e.g., concentrated glycerin).

The content of the isotonicity agent in the oil-in-water type emulsion of the present invention is such an amount that renders the osmotic pressure of the aqueous liquid preparation generally 200 - 400 mOsm, preferably 250 - 350 mOsm, further preferably 270 - 330 mOsm.

The oil-in-water type emulsion of the present invention can contain a water-soluble polymer to increase emulsion stability. Examples of the water-soluble polymer include povidone(polyvinylpyrrolidone), poly(vinyl alcohol), hydroxyethylcellulose, hydroxypropylcellulose, methylcellulose, hydroxypropylmethylcellulose, carboxymethylcellulose, and a salt thereof and the like.

The oil-in-water type emulsion of the present invention can contain a preservative. Examples of the preservative include quaternary ammonium salts such as benzalkonium chloride, benzethonium chloride and the like, cation compounds such as chlorhexidine gluconate and the like, para-hydroxybenzoate such as methyl para-hydroxybenzoate, propyl para-hydroxybenzoate and the like, alcohol compounds such as chlorobutanol, benzyl alcohol and the like, sodium dehydroacetate, thimerosal, sorbic acid and the like.

The oil-in-water type emulsion of the present invention can contain various other additives such as stabilizer, antioxidant, pH adjuster, thickener and the like. Examples of the antioxidant include ascorbic acid and a salt thereof, tocopherol, sodium thiosulfate, sodium bisulfite, pyruvic acid and a salt thereof and the like. Examples of the pH adjuster include hydrochloric acid, phosphoric acid, acetic acid, sulfuric acid, sodium hydroxide, potassium hydroxide, sodium carbonate, sodium hydrogen carbonate, aqueous ammonia and the like.

The pH of the oil-in-water type emulsion of the present invention is preferably 3 - 8, further preferably 4 - 6.

The median diameter of the oil drop in the oil-in-water type emulsion of the present invention is preferably 0.0001 - 5 $\mu$m, further more preferably 0.001 - 1 $\mu$m, particularly preferably 0.01 - 1 $\mu$m. The median diameter can be measured using a particle size distribution measuring apparatus.

The oil-in-water type emulsion of the present invention can be prepared using a known method. For example, the oil-in-water type emulsion of the present invention can be prepared by emulsifying an oil component, in which compound A or a salt thereof is dissolved, in water, in which ethylenediaminetetraacetic acid or a salt thereof or a hydrate thereof is dissolved, by using an emulsifier. For example, an emulsifier; ethylenediaminetetraacetic acid or a salt thereof or a hydrate thereof; and the above-mentioned additives as necessary are added to water, oil, in which compound A or a salt thereof is dissolved, is added and the mixture can be emulsified. To perform uniform emulsification, a known means such as homomixer, homogenizer, microfluidizer, high-pressure homogenizer and the like can be used.

When the aqueous liquid preparation of the present invention is an aqueous solution containing compound A or a salt thereof (hereinafter to be also referred to as the aqueous solution of the present invention), various additives generally used such as buffering agent, isotonicity agent, pH adjuster, surfactant and the like may be added as appropriate. As the buffering agent, isotonicity agent, and pH adjuster, those similar to the examples recited above for the oil-in-water type emulsion can be mentioned.

As the surfactant, nonionic surfactants can be mentioned. Examples of the nonionic surfactant include polyoxyethylene hydrogenated castor oils and polyoxyethylene sorbitan fatty acid ester, preferably sorbitan polyoxyethylene monooleates (e.g., polysorbate 80), polyoxyethylene sorbitan monolaurates, sorbitan polyoxyethylene monopalmitates, sorbitan polyoxyethylene monostearates and the like.

The content of the buffering agent in the aqueous solution of the present invention is generally 0.01 - 1 w/v%, preferably 0.02 - 0.5 w/v%, further preferably 0.05 - 0.2 w/v%, relative to the total amount of the aqueous liquid preparation.

The content of the isotonicity agent in the aqueous solution of the present invention is such an amount that renders the osmotic pressure of the aqueous liquid preparation generally 200 - 400 mOsm, preferably 250 - 350 mOsm, further preferably 270 - 330 mOsm.

The content of the surfactant in the aqueous solution of the present invention is generally 0.05 - 10 w/v% relative to the total amount of the aqueous liquid preparation.

The pH of the aqueous solution of the present invention is preferably 3 - 8, further preferably 4 - 6.

The aqueous solution of the present invention can be prepared by a known method, by dissolving compound A or a salt thereof; ethylenediaminetetraacetic acid or a salt thereof or a hydrate thereof; and the above-mentioned additives as necessary in water.

In the present invention, the amounts of the additives and each component according to the above-mentioned aqueous solution are applied to the suspension.

The aqueous liquid preparation of the present invention can be used as an agent for the prophylaxis or treatment of, for example, ophthalmic diseases such as diabetic retinopathy and the like in mammals (human, dog, rabbit, bovine, horse, monkey, cat, sheep etc.).

While the aqueous liquid preparation of the present invention can be formulated in the dosage form of an aqueous liquid preparation for oral administration or parenteral administration, when compound A or a salt thereof is used for ophthalmic diseases such as diabetic retinopathy and the like, eye drop is preferable.

While the dose of the aqueous liquid preparation of the present invention varies depending on the kind of disease, symptom, and age, body weight and the like of patients, for example, when it is used as an eye drop to an adult, an eye drop containing about 0.002 - 2 w/v% of compound A or a salt thereof is desirably administered by instillation of 1 - 2 drops per administration for one eye of a patient about 1 - 4 times per day according to the symptom.

The present invention also relates to a method of stabilizing compound A and a salt thereof in an aqueous liquid, which is characterized by adding compound A or a salt thereof, and ethylenediaminetetraacetic acid or a salt thereof or a hydrate thereof to an aqueous liquid.

The order of addition is not particularly limited. For example, compound A or a salt thereof can be added to an aqueous liquid, to which ethylenediaminetetraacetic acid or a salt thereof or a hydrate thereof is added in advance; or ethylenediaminetetraacetic acid or a salt thereof or a hydrate thereof, and compound A or a salt thereof can be simultaneously added to an aqueous liquid.

When the aqueous liquid is an oil-in-water type milky liquid, compound A or a salt thereof is dissolved in oil and then added.

Formulating in the method of the present invention is performed in an embodiment similar to that of an aqueous liquid preparation. For example, the amount of ethylenediaminetetraacetic acid or a salt thereof or a hydrate thereof follows the amounts indicated for the aqueous liquid preparation.

The method may include a step of confirming stabilization (e.g., a step of measuring the content of compound A or a salt thereof in an aqueous liquid, a step of measuring the residual ratio of compound A or a salt thereof in an aqueous liquid, a step of measuring the half-life of compound A or a salt thereof in an aqueous liquid etc.). The step of measuring the content, the step of measuring the residual ratio, and the step of measuring the half-life can be performed according to, for example, the below-mentioned Experimental Example 1.

The present invention further relates to a stabilizer of compound A and a salt thereof in an aqueous liquid, which contains ethylenediaminetetraacetic acid or a salt thereof or a hydrate thereof.

The stabilizer of the present invention contains at least ethylenediaminetetraacetic acid or a salt thereof or a hydrate thereof, and may further contain various additives such as buffering agent, isotonicity agent, pH adjuster, surfactant and the like.

The stabilizer can be used in an embodiment similar to that of formulating an aqueous liquid preparation.

The present invention further relates to a stabilizer of compound A and a salt thereof in an aqueous liquid, which contains ethylenediaminetetraacetic acid or a salt thereof or a hydrate thereof, and castor oil in combination.

The stabilizer exhibits a stabilizing effect of castor oil on the oil phase of an oil-in-water type emulsion, in addition to the stabilization by ethylenediaminetetraacetic acid or a salt thereof or a hydrate thereof.

[Examples]

While the present invention is explained more concretely in the following by referring to Examples, Comparative Examples, Experimental Examples, and Reference Examples, the present invention is not limited to them.

Compound A is N-[5-({2-[(cyclopropylcarbonyl)amino]imidazo[1,2-b]pyridazin-6-yl}oxy)-2-methylphenyl]-1,3-dimethyl-1H-pyrazole-5-carboxamide. The sodium edetate is ethylenediaminetetraacetic acid disodium salt dihydrate.

[Examples 1 and 2]

According to the formulation shown in Table 1, compound A, polysorbate 80, concentrated glycerin, sodium acetate, sodium edetate were added to and dissolved in purified water, and the mixture was adjusted to pH 5.5 with hydrochloric acid to give the aqueous liquid preparations of Examples 1 and 2 (compound A-containing aqueous solutions).

[Examples 3 and 4]

According to the formulation shown in Table 1, polysorbate 80, concentrated glycerin, sodium acetate, sodium edetate were added to and dissolved in purified water to give an aqueous phase. Separately, according to the formulation shown in Table 1, compound A was added to and dissolved in castor oil to give an oil phase. The oil phase was added while stirring the aqueous phase by T.K. ROBOMIX (manufactured by PRIMIX Corporation) to give a crude emulsion. The crude emulsion was processed into fine particles by FILMIX (manufactured by PRIMIX Corporation), and adjusted to pH 5.5 with hydrochloric acid to give aqueous liquid preparations (compound A-containing oil-in-water type emulsions) of Examples 3 and 4.

[Comparative Examples 1 - 4]

According to the formulation shown in Table 1 and in the same manner as in Examples 1 - 4 except that sodium edetate was not used, the aqueous liquid preparations (compound A-containing aqueous solutions) of Comparative Examples 1 and 2, and the aqueous liquid preparations (compound A-containing oil-in-water type emulsions) of Comparative Examples 3 and 4 were obtained.

Table 1

| | | | | | | | | (in 100 mL) |
|---|---|---|---|---|---|---|---|---|
| preparation | Comp. Ex. 1 | Comp. Ex. 2 | Ex. 1 | Ex. 2 | Comp. Ex. 3 | Comp. Ex. 4 | Ex. 3 | Ex. 4 |
| compound A | 0.005 g | 0.01 g | 0.005 g | 0.01 g | 0.005 g | 0.01 g | 0.005 g | 0.01 g |
| castor oil | - | - | - | - | 5 g | 5 g | 5 g | 5 g |
| polysorbate 80 | 10 g | 10 g | 10 g | 10 g | 4 g | 4 g | 4 g | 4 g |
| concentrated glycerin | 2.2 g | 2.2 g | 2.2 g | 2.2 g | 2.2 g | 2.2 g | 2.2 g | 2.2 g |
| sodium acetate | 0.05 g | 0.05 g | 0.05 g | 0.05 g | 0.05 g | 0.05 g | 0.05 g | 0.05 g |
| sodium edetate | - | - | 0.02 g | 0.02 g | - | - | 0.02 g | 0.02 g |
| hydrochloric acid | q.s. | q.s. | q.s. | q.s. | q.s. | q.s. | q.s. | q.s. |
| purified water | q.s. | q.s. | q.s. | q.s. | q.s. | q.s. | q.s. | q.s. |
| pH | 5.5 | 5.5 | 5.5 | 5.5 | 5.5 | 5.5 | 5.5 | 5.5 |

[Experimental Example 1] Stability test

The aqueous liquid preparations obtained in Examples 1 - 4 and Comparative Examples 1 - 4 were each filled in a glass ampoule, and stored at 60°C for 5 days, 7 days, 14 days. The content of compound A in the samples after preservation was measured by the following method.

(compound A content measurement method)

(1) Formulation of sample solution

Each sample (2 mL) after the above-mentioned storage was accurately measured, ethanol was added precisely to 20 mL.

(2) Formulation of standard solution

Compound A (about 0.02 g) was precisely measured, ethanol was added and the compound was dissolved accurately to 20 mL (standard stock solution). The standard stock solution was diluted with injector washing solution by the method shown in Table 2 to give standard solutions (STD-1 - STD-5).

Table 2

| standard solution preparation method | | | | | |
|---|---|---|---|---|---|
| measured solution | Standard stock solution | Standard stock solution | STD-1 | STD-1 | STD-2 |
| measured amount | 2 mL | 1 mL | 2 mL | 1 mL | 1 mL |
| diluting to | 20 mL | 20 mL | 10 mL | 10 mL | 10 mL |
| standard solution | STD-1 | STD-2 | STD-3 | STD-4 | STD-5 |

(3) Measurement of compound A by liquid chromatography method

The sample solution and standard solution (20 $\mu$L) were subjected to a test by a liquid chromatography method under the following conditions, and the peak area (AT) of compound A in the sample solution was obtained. Similarly, the peak areas (AS1 - AS5) of compound A in the standard solutions (STD-1 - STD-5) were obtained, a standard curve was plotted, and the content and residual ratio of compound A in the sample solution were calculated from the y section and slope of the standard curve.

i) content of compound A = (AT-y section)/slope
ii) residual ratio (%) = amount of compound A at each measurement time point (percent strength %)/initial amount of compound A (percent strength %)$\times$100
iii) HPLC measurement condition

detector: ultraviolet absorption spectrophotometer (measurement wavelength 230 nm)
column: CAPCELL PAK C18 MGII, 3 $\mu$m, 4.6 mm i.d.$\times$100 mm (manufactured by Shiseido Co., Ltd.)
column temperature: constant temperature near 40°C
mobile phase: 0.02 mol/L phosphate buffer (pH 5.0)/acetonitrile mixed solution (13:7)
flow: adjusted such that retention time of compound A was about 8 min

(4) Calculation of half-life of compound A

The number of days of storage of stored sample at 60°C was taken on the X axis, and the logarithmic values of the residual ratio of compound A in the samples stored at 60°C for 5 days, 7 days, 14 days were each taken on the Y axis, and the slope of the straight line connecting those points was calculated as the reaction rate constant (k). The half-life (t1/2) of compound A in the solution was calculated from k.

```
half-life (t1/2) of compound A in solution = ln2/k
```

The results are shown in Tables 3 - 6.
In the aqueous liquid preparation, a decrease in the content of compound A was observed over days. However, the ratio of decrease decreased by the addition of sodium edetate. Particularly, the half-life of compound A in the 0.005%, 0.01% aqueous solutions was extended 1.40-fold (Table 3) and 3.57-fold (Table 4), as compared to no addition of sodium edetate.
The stability of compound A was improved more in the oil-in-water type emulsion than in the aqueous solution. An extension effect of 12.9-fold (Table 5, comparison of Comparative Example 3 and Example 3) and 12.1-fold (Table 6, comparison of Comparative Example 4 and Example 4) in the half-life of compound A in 0.005%, 0.01% oil-in-water type emulsion was seen by the addition of sodium edetate as compared to no addition of sodium edetate.
It was found that the stabilizing effect of sodium edetate on compound A in the solution was remarkably higher in the oil-in-water type emulsion than in the aqueous solution.
In addition, the residual ratio of compound A in Example 4, which was preserved at 60°C for 14 days, was 97.3%, and compound A was stable in the preparation.

Table 3

| stabilizing effect of sodium edetate on aqueous solution containing 0.005% compound A | | |
|---|---|---|
| preparation | Comparative Example 1 | Example 1 |
| correlation coefficient | -0.9725 | -0.9624 |
| $k$ (day$^{-1}$) | 0.0394 | 0.0282 |
| $t_{1/2}$ (day) | 17.6 | 24.5 |
| $t_{1/2}$ ratio relative to Comparative Example 1 | 1.00 | 1.40 |

[Table 4]

| stabilizing effect of sodium edetate on aqueous solution containing 0.01% compound A | | |
|---|---|---|
| preparation | Comparative Example 2 | Example 2 |
| correlation coefficient | -0.9891 | -0.9847 |
| $k$ (day$^{-1}$) | 0.0313 | 0.0087 |
| $t_{1/2}$ (day) | 22.2 | 79.2 |
| $t_{1/2}$ ratio relative to Comparative Example 2 | 1.00 | 3.57 |

[Table 5]

| stabilizing effect of sodium edetate on oil-in-water type emulsion containing 0.005% compound A. | | |
|---|---|---|
| preparation | Comparative Example 3 | Example 3 |
| correlation coefficient | -0.9882 | -0.9927 |
| $k$ (day$^{-1}$) | 0.0351 | 0.0027 |
| $t_{1/2}$ (day) | 19.8 | 255.6 |
| ratio of $t_{1/2}$ relative to Comparative Example 1 | 1.12 | 14.52 |

[Table 6]

| stabilizing effect of sodium edetate on oil-in-water type emulsion containing 0.01% compound A | | |
|---|---|---|
| preparation | Comparative Example 4 | Example 4 |
| correlation coefficient | -0.9787 | -0.9561 |
| $k$ (day$^{-1}$) | 0.0229 | 0.0019 |
| $t_{1/2}$ (day) | 30.3 | 366.2 |
| ratio of $t_{1/2}$ relative to Comparative Example 2 | 1.37 | 16.51 |

[Reference Example 1] measurement of solubility of compound A in various solvents (oil components)

An excess amount (about 0.2 g) of compound A was added to various solvents (10 g) described in Table 7, and the mixture was stirred at 25°C for 24 hr. Thereafter, the solvent was filtered off through a 0.45 μm non-aqueous filter while maintaining the temperature. The content of compound A contained in the filtrate was measured in the same manner as in Experimental Example 1, and the solubility of compound A in various solvents was calculated.

The results are shown in Table 7.

[Table 7]

| solubility of compound A in various solvents | |
|---|---|
| solvent | solubility (%) |
| castor oil | 0.311 |
| olive oil | 0.009 |
| corn oil | 0.008 |
| cottonseed oil | 0.008 |
| rape seed oil | 0.007 |
| soybean oil | 0.007 |
| liquid paraffin | N.D. |
| N.D. shows not more than detection limit. | |

[Industrial Applicability]

The aqueous liquid preparation (e.g., eye drop) containing compound A or a salt thereof of the present invention improves the stability of compound A or a salt thereof in the aqueous liquid preparation, and is useful as a medicament.

This application is based on a patent application No. 2014-250377 filed in Japan, the contents of which are incorporated in full herein.

**Claims**

1. An aqueous liquid preparation comprising N-[5-({2-[(cyclopropylcarbonyl)amino]imidazo[1,2-b]pyridazin-6-yl}oxy)-2-methylphenyl]-1,3-dimethyl-1H-pyrazole-5-carboxamide or a salt thereof, and ethylenediaminetetraacetic acid or a salt thereof or a hydrate thereof.

2. The preparation according to claim 1, which is an oil-in-water type emulsion.

3. The preparation according to claim 2, wherein an oil component in the oil-in-water type emulsion is castor oil.

4. The preparation according to any one of claims 1 to 3, which is an eye drop.

5. A method of stabilizing N-[5-({2-[(cyclopropylcarbonyl)amino]imidazo[1,2-b]pyridazin-6-yl}oxy)-2-methylphenyl]-1,3-dimethyl-1H-pyrazole-5-carboxamide or a salt thereof in an aqueous liquid, comprising adding N-[5-({2-[(cyclopropylcarbonyl)amino]imidazo[1,2-b]pyridazin-6-yl}oxy)-2-methylphenyl]-1,3-dimethyl-1H-pyrazole-5-carboxamide or a salt thereof, and ethylenediaminetetraacetic acid or a salt thereof or a hydrate thereof to the aqueous liquid.

6. The method according to claim 5, wherein the aqueous liquid is an oil-in-water type milky liquid.

7. The method according to claim 6, wherein an oil component in the oil-in-water type milky liquid is castor oil.

8. A stabilizer of N-[5-({2-[(cyclopropylcarbonyl)amino]imidazo[1,2-b]pyridazin-6-yl}oxy)-2-methylphenyl]-1,3-dimethyl-1H-pyrazole-5-carboxamide or a salt thereof in an aqueous liquid, comprising ethylenediaminetetraacetic acid or a salt thereof or a hydrate thereof.

9. A stabilizer of N-[5-({2-[(cyclopropylcarbonyl)amino]imidazo[1,2-b]pyridazin-6-yl}oxy)-2-methylphenyl]-1,3-dimethyl-1H-pyrazole-5-carboxamide or a salt thereof in an aqueous liquid, comprising ethylenediaminetetraacetic acid or a salt thereof or a hydrate thereof, and castor oil in combination.

## INTERNATIONAL SEARCH REPORT

| | International application No. |
|---|---|
| | PCT/JP2015/084618 |

### A. CLASSIFICATION OF SUBJECT MATTER
*A61K31/5025*(2006.01)i, *A61K9/08*(2006.01)i, *A61K9/107*(2006.01)i, *A61K47/18*
(2006.01)i, *A61K47/44*(2006.01)i, *A61P3/10*(2006.01)i, *A61P9/10*(2006.01)i,
*A61P35/00*(2006.01)i, *A61P43/00*(2006.01)i

According to International Patent Classification (IPC) or to both national classification and IPC

### B. FIELDS SEARCHED

Minimum documentation searched (classification system followed by classification symbols)
A61K31/5025, A61K9/08, A61K9/107, A61K47/18, A61K47/44, A61P3/10, A61P9/10,
A61P35/00, A61P43/00

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN)

### C. DOCUMENTS CONSIDERED TO BE RELEVANT

| Category* | Citation of document, with indication, where appropriate, of the relevant passages | Relevant to claim No. |
|---|---|---|
| Y | WO 2008/016192 A2 (TAKEDA PHARMACEUTICAL CO., LTD.), 07 February 2008 (07.02.2008), description, page 139, 11th paragraph to page 140, 1st paragraph; examples & US 2009/0137595 A1 paragraph [0728]; examples & JP 2009-545583 A & EP 2049541 A2 & CA 2659971 A & KR 10-2009-0047509 A & CN 101541801 A | 1–9 |
| Y | JP 2009-73795 A (Lion Corp.), 09 April 2009 (09.04.2009), examples (Family: none) | 1–9 |

| ☒ | Further documents are listed in the continuation of Box C. | ☐ | See patent family annex. |
|---|---|---|---|

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 04 February 2016 (04.02.16) | 16 February 2016 (16.02.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | |
| | Telephone No. |

Form PCT/ISA/210 (second sheet) (January 2015)

**INTERNATIONAL SEARCH REPORT**

| International application No. |
| --- |
| PCT/JP2015/084618 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | WO 2005/021008 A1   (Hisamitsu Pharmaceutical Co., Inc.), 10 March 2005 (10.03.2005), examples & JP 4820647 B examples | 1-9 |
| Y | JP 2011-178688 A  (Sumika Enviro-Science Co., Ltd.), 15 September 2011 (15.09.2011), examples (Family: none) | 1-9 |
| Y | JP 2003-176228 A  (Rohto Pharmaceutical Co., Ltd.), 24 June 2003 (24.06.2003), examples (Family: none) | 1-9 |
| Y | JP 2008-518992 A  (Novagali Pharma SA), 05 June 2008 (05.06.2008), examples & US 2009/0028955 A1 examples & WO 2006/050837 A2     & EP 1655021 A1 & DE 602004017477 D     & CA 2578176 A & KR 10-2007-0083573 A   & CN 101014317 A | 1-9 |
| Y | WO 2014/074823 A1  (CLEARSIDE BIOMEDICAL, INC.), 15 May 2014 (15.05.2014), claims & US 2015/0258120 A1 claims; paragraph [0180] & EP 2916827 A1          & CA 2890471 A & CN 104884049 A         & KR 10-2015-0083117 A | 1-9 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008016192 A
- JP 2014250377 A